(19) Europäisches Patentamt
European Patent Office
Office européen des brevets

(11) Veröffentlichungsnummer: **0 414 219 B1**

(12) # EUROPÄISCHE PATENTSCHRIFT

(45) Veröffentlichungstag der Patentschrift: **18.10.95**

(51) Int. Cl.$^{6}$: **A61F 2/16**, A61L 27/00, G02B 1/04

(21) Anmeldenummer: **90116079.6**

(22) Anmeldetag: **22.08.90**

(54) **Verwendung von photopolymerisierbaren Massen als Intraokularlinsen-Füllmaterial bei der Bekämpfung von Katarakt und anderen Augenkrankheiten.**

(30) Priorität: **22.08.89 DE 3927667**

(43) Veröffentlichungstag der Anmeldung:
**27.02.91 Patentblatt 91/09**

(45) Bekanntmachung des Hinweises auf die Patenterteilung:
**18.10.95 Patentblatt 95/42**

(84) Benannte Vertragsstaaten:
**CH DE FR GB IT LI**

(56) Entgegenhaltungen:
**EP-A- 0 233 081**
**WO-A-89/00029**
**DE-C- 3 702 625**

**CHEMICAL ABSTRACTS, vol. 101, no. 24, 10 December 1984, Columbus, Ohio, US; abstract no. 212341R, MATSUSHITA ELECTRIC: 'PLASTIC LENSES' page 44 ;**

(73) Patentinhaber: **THERA Patent GmbH & Co. KG**
**Gesellschaft für industrielle Schutzrechte**
**Am Griesberg 2**
**D-82229 Seefeld (DE)**

(72) Erfinder: **Gasser, Oswald, Dr.**
**Höhenstrasse 10**
**D-8031 Seefeld (DE)**
Erfinder: **Wanek, Erich, Dr.**
**An der Breite 9**
**D-8031 Seefeld (DE)**
Erfinder: **Guggenberger, Rainer, Dr.**
**Siglstrasse 10**
**D-8036 Herrsching (DE)**
Erfinder: **Stefan, Klaus-Peter, Dr.**
**Hauptstrasse 36a**
**D-8031 Seefeld (DE)**
Erfinder: **Ellrich, Klaus, Dr.**
**Auinger Strasse 16**
**D-8031 Wörthsee (DE)**

(74) Vertreter: **Müller, Bernhard, Dr. H. Schmidt & B. Müller Patentanwälte**
**Graf-Toerring-Strasse 45**
**D-82229 Seefeld (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 414 219 B1

## Beschreibung

Die Erfindung betrifft die Verwendung von photopolymerisierbaren Zusammensetzungen zur Herstellung eines Intraokularlinsen-Füllmaterials, das intraoperativ bei eingesetzter Intraokularlinsenhülle angewandt wird. Die Behandlung von Katarakt (Grauer Star) sowie anderen Augenleiden erfordert häufig die Entfernung der natürlichen Linse des Auges. Seit der Implantation der ersten künstlichen Intraokularlinse (IOL) aus Polymethylmethacrylat (PMMA) (H. Ridley, 1948) wurde diese Operation in vielen ophtalmologischen Kliniken zur Routine.

Je nach Einsatzort unterscheidet man drei IOL-Typen: Vorderkammerlinsen, Intrapupillarlinsen und Hinterkammerlinsen. Die Linsen bestehen aus einem Linsenkörper und einer Haptik, die die Fixierung der Linse gewährleistet. Der Linsenkörper wird meist aus PMMA-Homopolymeren, seltener aus Copolymeren wie z.B. PMMA-Ethylenglykoldimethacrylat, PMMA-Hydroxyethylmethacrylat (HEMA) oder PMMA-Vinylpyrrolidon und seit neuerer Zeit aus Silikon-Elastomeren hergestellt. In vielen Fällen wird ein UV-Absorber zugefügt, um die Netzhaut vor UV-Licht zu schützen, wie das auch durch die natürliche Linse geschieht. Die Haptik kann sehr unterschiedlich geformt sein und besteht meistens aus Polypropylen, seltener aus PMMA.

Der gemeinsame Nachteil all dieser Systeme ist, daß es sich um Verhältnismäßig große, starre Körper handelt, so daß für die Implantation ein relativ großer Einschnitt durch das Hornhautendothel notwendig ist. Dieser Eingriff führt zur Schädigung und zum Verlust eines Teils der nicht regenerationsfähigen Hornhautendothelzellen. Mit der Größe des Einschnittes steigt außerdem die Gefahr der Bildung eines Hornhautödems, sowie der Entstehung von Astigmatismus. Ebenfalls nachteilig ist, daß jede IOL gesondert den speziellen Erfordernissen angepaßt werden muß, so daß sich die Notwendigkeit ergibt, eine Vielzahl von Typen unterschiedlicher Stärke zu produzieren und zu lagern.

Aus der EP-A-0308130 sind verformbare, elastische Intraokularlinsen bekannt, die hergestellt werden aus einer Mischung von monofunktionellen Acryl- bzw. Methacrylsäureestern unter Zusatz von geringen Mengen an difunktionellen Acryl- bzw. Methacrylsäureestern. Die Herstellung der Linsen erfolgt auf konventionelle Weise außerhalb des Körpers, wodurch eine Einstellung der Linsenstärke und der Brechungswerte während der Operation nicht möglich ist.

Aus DE-C-3702625 sind Intraokularlinsen zur Implantation nach einem extrakapsulären Katarakt bekannt, die aus einer elastischen Silikonhülle bestehen, die mit einer transparenten Füllung eines härtbaren Materials Versehen sind. Das härtbare Material soll aus "transparentem Acryl" bestehen, welchem ein Photoinitiator beigegeben ist. In dieser Patentschrift finden sich keine Angaben über die Natur der verwendeten Monomere bzw. Photoinitiatoren.

WO 89/00029 beschreibt die Verwendung von photopolymerisierbaren Zusammensetzungen zur Herstellung eines Intraokularlinsen-Füllmaterials, das intraoperativ nach der Entfernung der Augenlinse in den Kapselsack eingebracht wird, wobei die photopolymerisierbare Zusammensetzung einen (Meth)-acrylsäureester eines aliphatischen Präpolymers und einen Photoinitiator enthält. Ein Hinweis auf die Verwendung von (Meth)acrylsäureestern mit aromatischem Grundgerüst sowie auf die Möglichkeit, daß in derartigen Zusammensetzungen gegebenenfalls UV-Absorber und sonstige Hilfsstoffe wie Farbstoffe oder Aktivatoren für den Photoinitiator enthalten sein können, findet sich in dieser Druckschrift nicht.

Aufgabe der Erfindung war die Bereitstellung einer photopolymerisierbaren Masse, die während der Operation am Auge entweder direkt in den Kapselsack eingefüllt werden kann oder in eine vorher implantierte elastische Hülle ins Auge eingebracht werden kann. Da das Material über die Einspritzstelle nach dem Aushärten in direktem Kontakt mit dem Augeninnenraum ist, ist es nötig, daß das Intraokularlinsen-Füllmaterial höchstens geringfügig toxisch ist sowie keine größeren Mengen aus dem polymerisierten Material auslaugbar sind.

Gegenstand der Erfindung ist deswegen die Verwendung von photopolymerisierbaren Zusammensetzungen zur Herstellung eines Intraokularlinsen-Füllmaterials, das intraoperativ bei eingesetzter Intraokularlinsenhülle angewandt wird, dadurch gekennzeichnet, daß die photopolymerisierbaren Zusammensetzungen folgende Komponenten enthalten:

a) 90 - 99,99 Gew.-%, vorzugsweise 94 - 99,799 Gew.-%, mindestens eines mindestens difunktionellen Acryl- und/oder Methacrylsäureesters von mindestens difunktionellen Polyhydroxyverbindungen mit aliphatischem und/oder aromatischem Grundgerüst mit wenigstens 6 Kettengliedern, wobei das Grundgerüst aus den Atomen Kohlenstoff und Sauerstoff besteht,

b) 0,01 - 5 Gew.-%, vorzugsweise 0,1 - 2 Gew.-%, mindestens eines Photoinitiators, der mit Licht im Wellenlängenbereich 400-500 nm aktivierbar ist,

c) 0,001 - 9,98 Gew.-%, vorzugsweise 0,001 - 2 Gew.-%, eines UV-Absorbers, der Licht von Wellenlängen $< 400$ nm absorbieren kann, und

d) 0,01 - 9,98 Gew.-%, vorzugsweise 0,1 - 2 Gew.-%, sonstiger Hilfsstoffe, wie Farbstoffe oder Aktivatoren für den Photoinitiator, z.B. tertiäre Amine,
wobei sich die Mengenangaben jeweils auf die Gesamtmasse beziehen.

Gegenstand der Erfindung ist ferner die Verwendung von photopolymerisierbaren Zusammensetzungen zur Herstellung eines Intraocularlinsen-Füllmaterials, das intraoperativ bei eingesetzter Intraocularlinsenhülle angewandt wird, dadurch gekennzeichnet, daß die photopolymerisierbaren Zusammensetzungen folgende Komponenten enthalten:

a) 90 - 99,99 Gew.-%, vorzugsweise 94 - 99,799 Gew.-%, mindestens eines mindestens difunktionellen Acryl- bzw. Methacrylsäureesters von Bisphenolen,
b) 0,01 - 5 Gew.-%, vorzugsweise 0,1 - 2 Gew.-%, mindestens eines Photoinitiators, der mit Licht im Wellenlängenbereich 400-500 nm aktivierbar ist,
c) 0 - 9,98 Gew.-%, vorzugsweise 0,001 - 2 Gew.-%, eines UV-Absorbers, der Licht von Wellenlängen < 400 nm absorbieren kann, und
d) 0 - 9,98 Gew.-%, vorzugsweise 0,1 - 2 Gew.-%, sonstiger Hilfsstoffe, wie Farbstoffe oder Aktivatoren für den Photoinitiator, z.B. tertiäre Amine,
wobei sich die Mengenangaben jeweils auf die Gesamtmasse beziehen.

Die mindestens difunktionellen Acryl- bzw. Methacrylsäureester haben vorzugsweise ein Molekulargewicht > 310 und sind vorzugsweise die Diacryl- bzw. Dimethacrylsäureester mindestens difunktioneller Polyhydroxyverbindungen mit aliphatischem und/oder aromatischem Grundgerüst mit wenigstens 6 Kettengliedern, wobei das Grundgerüst aus den Atomen Kohlenstoff, Sauerstoff, Stickstoff besteht.

Gut einsetzbar sind beispielsweise die Diacryl- bzw. Dimethacrylsäureester von Bisphenolen, z. B. Bisphenol A bzw. die mit Ethylenoxid bzw. Propylenoxid verlängerten Bishydroxy-polyalkyloxy-bisphenol-A-Derivate. Bevorzugt sind hierbei die beidseitig mit 1 bis 5 und insbesondere mit 1 - 3 Ethylenoxideinheiten verlängerten Bisphenol A-Typen. Besonders bevorzugt sind Verbindungen der Formel I.

Formel I: 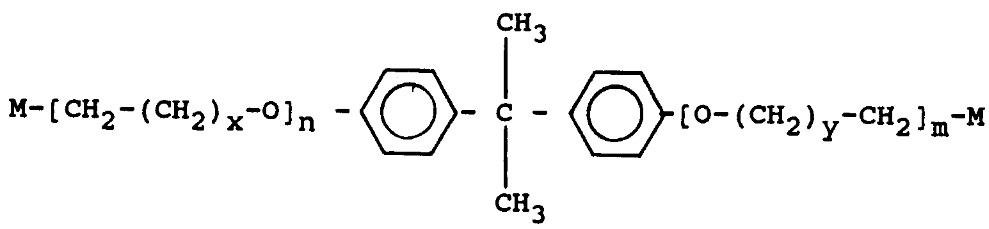

wobei M =

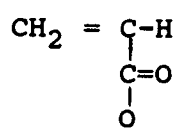 bzw. 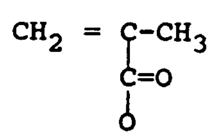

und n, m = 1-5, insbesondere 1-3
und x, y = 1, 2, 3, insbesondere 1.

Gut einsetzbar sind auch die Bisacrylsäure- bzw. Bismethacrylsäureester cycloaliphatischer Diole insbesondere die mit Ethylenoxid und Propylenoxid verlängerten cycloaliphatischen Diole. Besonders geeignet sind die Diole des mit Ethylenoxid verlängerten Bis-(hydroxymethyl)-tricyclo[5.2.1.$0^{2,6}$]-decans, Vorzugsweise ist jede OH-Gruppe mit 1 - 5 und insbesondere mit 1 - 3 Ethylenoxideinheiten verlängert.

Gut einsetzbare mindestens difunktionelle Acrylsäure- bzw. Methacylsäureester sind auch die Umsetzungsprodukte von mindestens 2 Teilen Hydroxyalkylacrylat bzw. Hydroxyalkylmethacrylat mit mindestens difunktionellen Polyisocyanaten mit aliphatischem bzw. aromatischem Grundgerüst. Besonders bevorzugt sind die Umsetzungsprodukte von 2 Teilen Hydroxyalkylmethacrylaten z. B. Hydroxypropylmethacrylat mit 1 Teil Trimethylhexamethylendiisocyanat.

Zur Einstellung der Fließeigenschaften, Viskosität sowie optischen Eigenschaften ist es möglich, verschiedene mindest difunktionelle Acrylsäure- bzw. Methacrylsäureester miteinander zu mischen.

Als Komponente 2 eingesetzt werden können Photoinitiatoren, die Licht im Bereich 400 - 500 nm absorbieren, wie z. B. Alpha-diketone, wie sie aus DE-A-2302820 und DE-A-2251045 bekannt sind, insbesondere Campherchinon, Benzil- und Phenanthrenchinon, sowie Mono- und Bisacylphosphinoxide, wie

sie aus der EP-A-0184094 und der EP-A-73413, EP-A-7508 und EP-A-57474 bekannt sind, besonders bevorzugt sind beispielsweise 2,4,6-Trimethylbenzoyl-diphenylphosphinoxid und Bis-(2,6-dichlorbenzoyl)-4-n-propylphenylphosphinoxid bzw. Bis-(2,6-dichlorbenzoyl)-4-n-butylphenylphosphinoxid.

Zur Beschleunigung der Polymerisation sowie zur Verringerung der Schmierschicht und der Restmonomeren kann es sinnvoll sein, zusammen mit den Photoinitiatoren sogenannte Aktivatoren einzusetzen. Hierzu sind insbesondere tertiäre Amine gut geeignet wie Trihexylamin, Triethylamin, Triethanolamin und ähnliche. Besonders gut geeignet sind auch die mit Methacrylsäure- bzw. Acrylsäureester umgesetzten Polyhydroxyalkylamine.

Um die Netzhaut vor einfallender UV-Strahlung zu schützen, kann es sinnvoll sein, die photopolymerisierbaren Zusammensetzungen mit sogenannten UV-Absorbern zu versehen. Besonders gut geeignet sind UV-Absorber, die eine Absorption des Wellenlängenbereichs $< 400$ nm möglichst vollständig gewährleisten, ohne die für die Polymerisation nötige Wellenlänge $> 400$ nm herauszufiltern. Gut geeignet sind z. B. Benzophenone, die Substituenten wie Hydroxyl- und/oder Alkoxylgruppen in 2 und/oder 4-Stellung enthalten, desweiteren substituierte Benzotriazole sowie in 3-Stellung phenylsubstituierte Acrylate, gegebenenfalls mit Cyanogruppen in 2-Stellung sowie Salicylate. Gut geeignet sind insbesondere die UV-Absorber, die im Molekül eingebaute einpolymerisierbare Gruppen, wie z. B. Acrylsäure-bzw. Methacrylsäureester enthalten.

Weiterhin kann es sinnvoll sein, zur Einstellung der Viskosität bzw. des Brechungswertes organische und/oder anorganische Füllstoffe einzusetzen. Anorganische Füllstoffe sind beispielsweise pyrogenes Siliciumdioxid, welches vorzugsweise mit Silanen oberflächenbehandelt wurde. Bei Einsatz von anorganischen Füllstoffen ist allerdings darauf zu achten, daß der Brechungsindex mit den anderen Komponenten übereinstimmt, so daß keine Trübung der Linse auftritt. Zur Einstellung einer gewissen Tixotrophie ist es möglich, 0,5 - 3 Gew.-% silanisierter pyrogener Kieselsäure einzusetzen. Organische Füllstoffe können z. B. polymere Füllstoffe sein, die löslich oder unlöslich in den verwendeten Monomeren sein können. Bei unlöslichen Polymeren sollte der Brechungsindex des Polymeres mit dem der Monomeren nach der Polymerisation soweit übereinstimmen, daß keine Trübung der polymerisierten Linse auftreten kann. Lösliche Polymere können beispielsweise Polymethylmethacrylat u. ä. sein. Sie dienen zum Einstellen der Viskosität bzw. des Brechungsindexes des Polymeren.

Sollte das Intraokularlinsen-Füllungsmaterial in eine vorher implantierte dünne, faltbare, elastische Kunststoffhülle eingefüllt werden, so sollten Brechungsindex des polymerisierten Intraokularlinsen-Füllungsmaterials und der Kunststoffhülle ebenfalls weitestgehend übereinstimmen, um keine Brechungsphänomene am Übergang Hülle/Füllung zu erzeugen.

Die Herstellung des Intraokularlinsen-Füllungsmaterials erfolgt dadurch, daß die zu verwendenden Monomere mit den Initiatoren sowie gegebenenfalls zugesetzten Aktivatoren, UV-Absorbern und/ oder Füllstoffen solange gerührt werden, bis eine homogene klare Mischung entsteht. Diese kann dann in die Applizierbehältnisse abgefüllt werden; besonders vorteilhaft ist, wenn vor der Abfüllung eine Sterilfiltration durchgeführt wird. Es ist aber auch möglich, eine Sterilisierung nach Abfüllung durch z. B. thermische Behandlung durchzuführen.

Bei Verwendung der erfindungsgemäßen Intraokularlinsen-Füllungsmaterialien geht der behandelnde Arzt vorteilhaft nach folgendem Verfahren vor:

Nach Eröffnung der Linsenkapsel durch Diszision wird die Vorderkammer durch einen schmalen Lanzenschnitt am oberen oder schläfenwärtigen Hornhautrand eröffnet. Die Linsenmasse wird exprimiert, ausgelöffelt oder mittels Kryostab ausgefroren oder mit einer Spül-Saugspritze entfernt. Eine Methode, die die Entfernung der Linse über einen sehr kleinen Inzisionsschnitt erlaubt, ist die Phakoemulsifation (F. Hollwich, Augenheilkunde, Thieme Verlag Stuttgart 1979, S. 133 - 137). Die Linse kann intrakapsulär, also gemeinsam mit der Kapsel, oder extrakapsulär entfernt werden.

Im Falle einer intrakapsulären Kataraktextraktion muß die Kapsel durch eine dünne, faltbare, elastische Kunststoffhülle (z. B. Silicon) ersetzt werden, in welche das Intraokularlinsen-Füll.material eingefüllt wird. Im Falle der extrakapsulären Extraktion kann der praktisch intakte Kapselsack die Rolle der Siliconhülle übernehmen. Diese Methode schließt jedoch die zusätzliche Verwendung der Kunststoffhülle nicht aus. Die anschließende Erhärtung des Füllungsmaterials erfolgt dann mit Hilfe von Licht im Wellenlängenbereich 400 - 500 nm durch Belichtung durch die erweiterte Pupille.

Die Vorteile, die sich bei Verwendung der erfindungsgemäßen Intraokular-Füllmaterialien ergeben, sind somit

- geringstmögliche Beschädigung des natürlichen Auges
- Herstellung der individuellen Linse während der Operation
- Einstellung der Brechungswerte während der Operation
- geringe Toxizität
- geringe Temperaturentwicklung bei der Aushärtung

- gute Retention in der Kapsel bzw. Kunststoffhülle durch geringen Schrumpf sowie
- geringer Gehalt an Restmonomeren und auslaugbaren Substanzen.

Nachstehend wird die Erfindung anhand von Beispielen näher erläutert.

Beispiele 1- 7

Intraokular-Füllmaterialien werden hergestellt durch Mischen der in der Tabelle 1 angegebenen Monomere mit den in der Tabelle angegebenen Gehalten (Gew.-%) an Initiatoren, Aktivatoren, UV-Absorbern. Es wird solange unter leichter Erwärmung gerührt, bis jeweils eine klare Lösung entsteht. Die Bestimmung der durchpolymerisierten Schichtdicke erfolgt durch Einfüllen in Kunststoffzylinder (Ø 8 mm, h 20 mm) und 20 sek. Bestrahlung mit einer dentalen Bestrahlungseinheit (Elipar 2, Firma Espe, Wellenlängenbereich 400 - 500 nm). Die Bestimmung der Schmierschicht erfolgt durch Einfüllen des Materials in einen Zylinder aus Kunststoff (Ø 15 mm, h 1,5 mm) und ebenfalls 20 sek. Bestrahlen mit Elipar 2). Anschließend wird die Scheibe gewogen, darauf mit einem Isopropanol-feuchten Tuch die Schmierschicht von der Oberfläche entfernt, mit Preßluft trockengeblasen und erneut gewogen.

Für die Restmonomerbestimmung werden zwischen zwei Objektträgern Prüfkörper zur Schmierschichtbestimmung, wie oben beschrieben wurde, hergestellt. Diese Werden zu einem Pulver mit einer Korngröße < 200 µm gemahlen. Aus dem Pulver wird eine 10 %ige Methylenchloridlösung hergestellt, welche 3 - 5 Min. im Ultraschallbad behandelt, zentrifugiert und eventuell filtriert wird. Die Auswertung erfolgt über die externe Standardmethode mittels HPLC.

Tabelle 1

| Nr. | Monomere | UV-Absorber [Gew.-%] | Initiatoren [Gew.-%] | Aktivatoren [Gew.-%] | Schichtdicke [mm] | Restmonomere [Gew.-%] | Schmierschicht [mg/cm$^2$] |
|---|---|---|---|---|---|---|---|
| 1 | Bis-(acryloyl-oxyethyl-oxyethyl-oxymethyl)-tricyclo-[5.2.1.0$^{0,6}$] decan | — | 2,4,6 Trimethyl-benzoyl-diphenyl-phosphin-oxid (0,5 %) | — | 7 | 0,5 | 0,3 |
| 2 | Bis-(acryloyl-oxyethyl-oxyethyl-oxymethyl)-tricyclo-[5.2.1.0$^{0,6}$] decan | 2-Hydroxy-4-methoxy-benzo-phenon (0,1 %) | Bis-(2,6 dichlor-benzoyl)-4-n-propylphenylphos-phinoxid (0,5 %) | — | 12,5 | 0,2 | 0,3 |
| 3 | 2,2-Bis[4-(acryloyl-oxyethyl-oxyethyl-oxy)phenyl]propan | — | Campherchinon (0,22 %) + 2,4,6 Trimethyl-benzoyl-diphenyl-phosphinoxid (0,5 %) | Methyl-di-ethanolamin (1 %) | 11 | 0,2 | 2 |
| 4 | 2,2-Bis[4-(acryloyl-oxyethyl-oxyethyl-oxy)phenyl]propan | --- | Bis(2,6 dichlor-benzoyl)-4-n-pro-pylphenylphosphin-oxid (0,5 %) | — | 7 | 0,4 | 2 |
| 5 | 2,2-Bis[4-(acryloyl-oxyethyl-oxyethyl-oxy)phenyl]-propan | 2-Hydroxy-4-methoxy-benzo-phenon (0,1 %) | Bis(2,6 dichlor-benzoyl)-4-n-pro-pylphenylphosphin-oxid (0,5 %) | — | 9 | 0,4 | 2 |

Forts. von Tabelle I

| | | | | | | | |
|---|---|---|---|---|---|---|---|
| 6 | 2,2-Bis[4-(methacryloyl-oxyethyl-oxy-ethyl-oxy)phenyl]propan | — | Bis(2,6 dichlorbenzoyl)-4-n-butylphenylphosphinoxid (0,5 %) | — | 7,2 | 0,4 | 4 |
| 7 | 2,2-Bis[methacryloyl-oxyethyl-oxy-ethyl-oxy)phenyl]propan | 2-Hydroxy-4-methoxy-benzophenon (0,1 %) | Bis(2,6 dichlorbenzoyl)-4-n-propylphenylphosphinoxid (0,5 %) | — | 7.0 | 0,6 | 5 |

Mit allen Massen ließen sich Siliconhüllen, wie sie für faltbare Intraokularlinsen einsetzbar sind, einwandfrei füllen; die Schmierschicht war so gering, daß die so gehärtete Füllung nicht ohne Zerstörung der Hülle entfernt werden konnte; bei Verwendung von monofunktionellen Acryl- bzw. Methacrylsäureestern ist die Schmierschicht so groß, daß keine stabile Füllung in der Intraokularlinse hergestellt werden kann.

Mit den Mischungen der Beispiele 4, 5, 6 und 7 wurden Zelltoxizitätsuntersuchungen durchgeführt, indem zwischen zwei Objektträgern die Füllungsmaterialien auspolymerisiert wurden (20 sek. Bestrahlung mit Elipar 2). Die so gefertigten Polymerscheiben wurden mit einem nichtzelltoxischen TCAB (= Tissue culture adhesive systeme for biomaterials) unter sterilen Bedingungen fixiert und in Objektträgerkulturflaschen eingebracht. Die Präparate wurden anschließend mit bovinen Endothelzellen bezüchtet sowie mit Epithelzellen unter der Anwesenheit von $^{3}H$ TdR. Die Inkubationszeit betrug 24 h. Bei der Auswertung wurde die Cytoplasmaausbreitung, das Fehlen von Zellkernen sowie das Vernetzen der Zellen begutachtet. Bei allen Proben zeigte sich, daß die Kern- und Cytoplasmastrukturen zu 95 % aufrecht erhalten waren und lediglich eine leichtfügige Vernetzung der Zellen zu beobachten war. Die Materialien sind somit als nur geringfügig toxisch einzustufen. Im Anschluß wurde das Monomere des Beispiels 7 unpolymerisiert auf Zelltoxizität untersucht. Hierbei stellte sich das gleiche Erscheinungsbild heraus, auch das unpolymerisierte Monomer zeigte nur mäßig toxische Eigenschaften.

Die erfindungsgemäßen Intraokularlinsen-Füllmaterialien sind somit für den Anwendungszweck sehr gut geeignet, da sie zum einen nur eine geringe Sauerstoffinhibition bei der Polymerisation aufweisen, geringe Restmonomergehalte haben und nur als mäßig toxisch einzustufen sind.

**Patentansprüche**

**1.** Verwendung von photopolymerisierbaren Zusammensetzungen zur Herstellung eines Intraocularlinsen-Füllmaterials, das intraoperativ bei eingesetzter Intraocularlinsenhülle angewandt wird, dadurch gekennzeichnet, daß die photopolymerisierbaren Zusammensetzungen folgende Komponenten enthalten:

a) 90 - 99,99 Gew.-%, vorzugsweise 94 - 99,799 Gew.-%, mindestens eines mindestens difunktionellen Acryl- und/oder Methacrylsäureesters von mindestens difunktionellen Polyhydroxyverbindungen mit aliphatischem und/oder aromatischem Grundgerüst mit wenigstens 6 Kettengliedern, wobei das Grundgerüst aus den Atomen Kohlenstoff und Sauerstoff besteht,

b) 0,01 - 5 Gew.-%, vorzugsweise 0,1 - 2 Gew.-%, mindestens eines Photoinitiators, der mit Licht im Wellenlängenbereich 400-500 nm aktivierbar ist,

c) 0,001 - 9,98 Gew.-%, vorzugsweise 0,001 - 2 Gew.-%, eines UV-Absorbers, der Licht von Wellenlängen < 400 nm absorbieren kann, und

d) 0,01 - 9,98 Gew.-%, vorzugsweise 0,1 - 2 Gew.-%, sonstiger Hilfsstoffe, wie Farbstoffe oder Aktivatoren für den Photoinitiator, z.B. tertiäre Amine,

wobei sich die Mengenangaben jeweils auf die Gesamtmasse beziehen.

**2.** Verwendung nach Anspruch 1, dadurch gekennzeichnet, daß als Komponente a. difunktionelle Acryl- bzw. Methacrylsäureester mit einem Molekulargewicht > 310 verwendet werden.

**3.** Verwendung von photopolymerisierbaren Zusammensetzungen zur Herstellung eines Intraocularlinsen-Füllmaterials, das intraoperativ bei eingesetzter Intraocularlinsenhülle angewandt wird, dadurch gekennzeichnet, daß die photopolymerisierbaren Zusammensetzungen folgende Komponenten enthalten:

a) 90 - 99,99 Gew.-%, vorzugsweise 94 - 99,799 Gew.-%, mindestens eines mindestens difunktionellen Acryl- bzw. Methacrylsäureesters von Bisphenolen.

b) 0,01 - 5 Gew.-%, vorzugsweise 0,1 - 2 Gew.-%, mindestens eines Photoinitiators, der mit Licht im Wellenlängenbereich 400-500 nm aktivierbar ist,

c) 0 - 9,98 Gew.-%, vorzugsweise 0,001 - 2 Gew.-%, eines UV-Absorbers, der Licht von Wellenlängen < 400 nm absorbieren kann, und

d) 0 - 9,98 Gew.-%, vorzugsweise 0,1 - 2 Gew.-%, sonstiger Hilfsstoffe, wie Farbstoffe oder Aktivatoren für den Photoinitiator, z.B. tertiäre Amine,

wobei sich die Mengenangaben jeweils auf die Gesamtmasse beziehen.

**4.** Verwendung nach Anspruch 3, dadurch gekennzeichnet, daß als Komponente a. die Diacryl- bzw. Dimethacrylsäureester von Bisphenol A bzw. die mit Ethylenoxid bzw. Propylenoxid verlängerten Bishydroxypolyalkyloxy-bisphenol-A-Derivate verwendet werden.

**5.** Verwendung nach Anspruch 4, dadurch gekennzeichnet, daß als Komponente a. eine Verbindung der allgemeinen Formel I verwendet wird

Formel I : 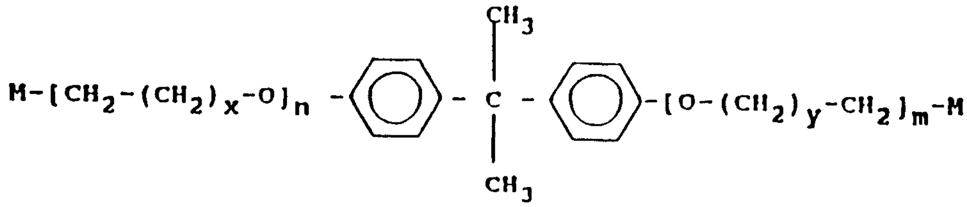

wobei M =

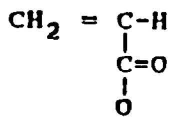 bzw. 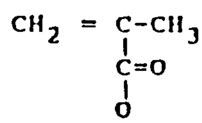

und n, m = 1-5, insbesondere 1-3
und x, y = 1, 2, 3, insbesondere 1.

**6.** Verwendung nach Anspruch 1, dadurch gekennzeichnet, daß als Komponente a. die Bisacrylsäure- bzw. Bismethacrylsäureester cycloaliphatischer Diole oder die entsprechenden Derivate, bei denen die Hydroxylgruppen mit 1 - 5 und insbesondere mit 1 - 3 Ethylenoxid- oder Propylenoxideinheiten verlängert sind, verwendet werden.

**7.** Verwendung nach Anspruch 6, dadurch gekennzeichnet, daß als Komponente a. die Bisacrylsäure- bzw. Bismethacrylsäureester der Diole des mit Ethylenoxid verlängerten Bis-(hydroxymethyl)-tricyclo-[5.2.1.$0^{2,6}$]-decans verwendet werden, wobei jede Hydroxylgruppe mit 1 - 5 und vorzugsweise mit 1 - 3 Ethylenoxid-Einheiten verlängert ist.

**8.** Verwendung nach Anspruch 1 oder 3, dadurch gekennzeichnet, daß als Komponente a. die Umsetzungsprodukte von mindestens zwei Teilen Hydroxyalkylacrylat bzw. Hydroxyalkylmethacrylat mit mindestens difunktionellen Polyisocyanaten mit aliphatischem, bzw. aromatischem Grundgerüst verwendet werden.

**9.** Verwendung nach Anspruch 1 oder 3, dadurch gekennzeichnet, daß als Komponente b. Alpha-diketone und/oder Mono- und/oder Bisacylphosphinoxide verwendet werden.

**Claims**

**1.** Use of photopolymerizable compositions for preparing an intraocular-lens filling material which is used during the operation with inserted intraocular lens shell, characterized in that the photopolymerizable compositions contain the following components:

a) 90-99,99 % by weight, preferably 94-99,799 % by weight, of at least one at least difunctional acrylic and/or methacrylic acid ester of at least difunctional polyhydroxy compounds with aliphatic and/or aromatic skeleton having at least six chain links, the skeleton consisting of the atoms carbon, oxygen, nitrogen,

b) 0,01-5 % by weight, preferably 0,1-2 % by weight, of at least one photoinitiator which is activatable with light in the wavelength range 400-500 nm,

c) 0,001-9,98 % by weight, preferably 0,001-2 % by weight, of a UV-absorber which can absorb light of wavelengths $< 400$ nm and

d) 0,001-9,98 % by weight, preferably 0,1-2 % by weight, of other auxiliary substances, such as dyes or activators for the photoinitiator, for example tertiary amines,

the quantity particulars referring in each case to the total mass.

**2.** Use according to claim 1, characterized in that as component a) difunctional acrylic or methacrylic acid esters having a molecular weight $> 310$ are used.

3. Use of photopolymerizable compositions for preparing an intraocular-lens filling material which is used during the operation with inserted intraocular lens shell, characterized in that the photopolymerizable compositions contain the following components:
   a) 90-99,99 % by weight, preferably 94-99,799 % by weight, of at least one at least difunctional acrylic and/or methacrylic acid ester of bisphenols,
   b) 0,01-5 % by weight, preferably 0,1-2 % by weight, of at least one photoinitiator which is activatable with light in the wavelength range 400-500 nm,
   c) 0-9,98 % by weight, preferably 0,001-2 % by weight, of a UV-absorber which can absorb light of wavelengths < 400 nm and
   d) 0-9,98 % by weight, preferably 0,1-2 % by weight, of other auxiliary substances, such as dyes or activators for the photoinitiator, for example tertiary amines,
   the quantity particulars referring in each case to the total mass.

4. Use according to claim 3, characterized in that as component a) the diacrylic or dimethacrylic acid esters of bisphenol A or the bishydroxy polyalkoxy bisphenol A derivatives lengthened with ethylene oxide or propylene oxide are used.

5. Use according to claim 4, characterized in that as component a) a compound of the general formula I is used

Formula I:

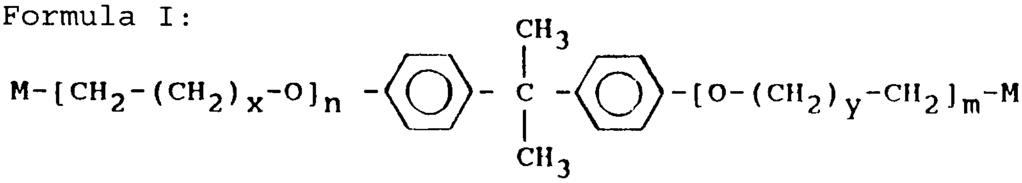

wherein M =

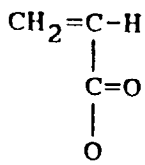

or

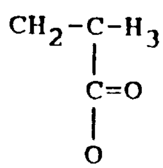

and n, m = 1-5, in particular 1-3,
and x, y = 1, 2, 3, in particular 1.

6. Use according to claim 1, characterized in that as component a) the bisacrylic acid or bismethacrylic acid esters of cycloaliphatic diols or the corresponding derivatives in which the hydroxyl groups are lengthened with 1-5 and in particular 1-3 ethylene oxide or propylene oxide units are used.

7. Use according to claim 6, characterized in that as component a) the bisacrylic acid or bismethacrylic acid esters of the diols of bis-(hydroxymethyl)-tricyclo[5.2.1.$0^{2,6}$] decane lengthened with ethylene oxide are used, each hydroxyl group being lengthened with 1-5 and preferably with 1-3 ethylene oxide units.

8. Use according to claim 1 or 3, characterized in that as component a) the reaction products of at least 2 parts hydroxyalkyl acrylate or hydroxyalkyl methacrylate with at least difunctional polyisocyanates having aliphatic or aromatic skeleton are used.

9. Use according to claim 1 or 3, characterized in that as component b) alpha-diketones and/or mono and/or bisacylphosphine oxides are used.

## Revendications

1. Utilisation de compositions photopolymérisables pour la production d'un matériau de remplissage des lentilles intra-oculaires ou cristallins, utilisées en cours d'opération sur la poche du cristallin en place, caractérisée en ce que la composition photopolymérisable contient les composants suivants :
   a) 90 - 99,99 % en poids, de préférence 94 - 99,799 % en poids, d'au moins un ester de l'acide acrylique et/ou méthacrylique difonctionnel d'au moins des composés polyhydroxy difonctionnels avec une structure de base aliphatique et/ou aromatique avec au moins 6 éléments de chaîne, la structure de base étant constituée par les atomes carbone et oxygène,
   b) 0,01 - 5 % en poids, de préférence 0,1 - 2 % en poids, d'au moins un photo-initiateur, qui peut être activé par la lumière dans la plage de longueurs d'ondes de 400-500 nm,
   c) 0,001 - 9,98 % en poids, de préférence 0,001 - 2 % en poids, d'un agent absorbant les UV, pouvant absorber la lumière des longueurs d'ondes < 400 nm, et
   d) 0,01 - 9,98 % en poids, de préférence 0,1 - 2 % en poids, d'autres matières auxiliaires telles que des colorants ou activateurs pour le photo-initiateur, par exemple des amines tertiaires,
   les indications de quantités étant chaque fois rapportées à la masse globale.

2. Utilisation selon la revendication 1, caractérisée en ce qu'en tant que composant a. on utilise des esters de l'acide acrylique ou méthacrylique difonctionnel ayant un poids moléculaire > 310.

3. Utilisation de compositions photopolymérisables pour la production d'un matériau de remplissage dans des lentilles intra-oculaires ou cristallins qui sont utilisées en cours d'opération sur la poche du cristallin en place, caractérisée en ce que les compositions photopolymérisables contiennent les composants suivants :
   a) 90 - 99,99 % en poids, de préférence 94 - 99,799 % en poids, d'au moins un ester de l'acide acrylique ou méthacrylique au moins difonctionnel des bisphénols,
   b) 0,01 - 5 % en poids, de préférence 0,1 - 2 % en poids, au moins d'un photo-initiateur qui peut être activé par la lumière dans une plage de longueurs d'ondes de 400-500 nm,
   c) 0 - 9,98 % en poids, de préférence 0,001 - 2 % en poids, d'un agent absorbant les UV, qui peut absorber la lumière des longueurs d'ondes < 400 nm, et
   d) 0 - 9,98 % en poids, de préférence 0,1 - 2 % en poids, d'autres matières auxiliaires telles que des colorants ou activateurs pour le photo-initiateur, par exemple des amines tertiaires,
   les indications de quantités se rapportant chaque fois à la masse globale.

4. Utilisation selon la revendication 3, caractérisée en ce qu'en tant que composant a. on utilise les esters de l'acide diacrylique ou diméthacrylique de bisphénol A ou les dérivés de bishydroxypolyalkyloxy-bisphénol A allongés par oxyde d'éthylène ou oxyde de propylène.

5. Utilisation selon la revendication 4, caractérisée en ce qu'en tant que composant a. on utilise un composé de la formule générale I

Formule I : 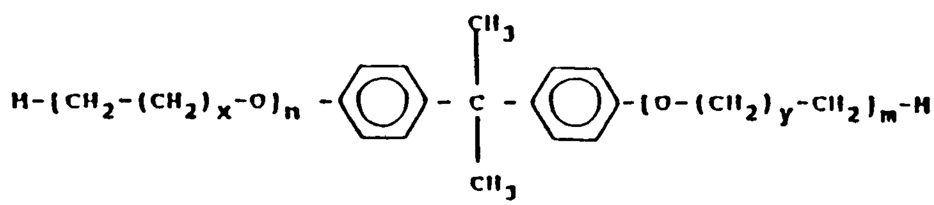

dans laquelle M =

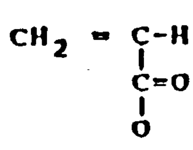 ou 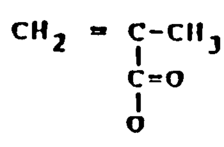

et n, m = 1-5, en particulier 1-3

et x, y = 1, 2, 3, en particulier 1.

**6.** Utilisation selon la revendication 1, caractérisée en ce qu'en tant que composant a. on utilise les esters de l'acide bisacrylique ou bis-méthacrylique de diols cycloaliphatiques ou les dérivés correspondants, dans lesquels on utilise les groupes hydroxyle allongés par 1-5 et en particulier 1-3 oxyde d'éthylène ou unités d'oxyde de propylène.

**7.** Utilisation selon la revendication 6, caractérisée en ce qu'en tant que composant a. on utilise les esters de l'acide bisacrylique ou bis-méthacrylique des diols de bis-(hydroxyméthyl)-tricyclo[5.2.1.$0^{2,6}$]-décane allongé par oxyde d'éthylène, chaque groupe hydroxyle étant allongé par 1 - 5 et de préférence par 1 - 3 unités d'oxyde d'éthylène.

**8.** Utilisation selon la revendication 1 ou 3, caractérisée en ce qu'en tant que composant a. on utilise les produits réactionnels d'au moins deux parties d'hydroxyalkylacrylate ou d'hydroxyalkylméthacrylate avec au moins des polyisocyanates difonctionnels avec une structure de base aliphatique ou aromatique.

**9.** Utilisation selon la revendication 1 ou 3, caractérisée en ce qu'en tant que composant b. on utilise des alpha-dicétones et/ou des mono- et/ou bisacylphosphinoxydes.